# EUROPÄISCHE PATENTSCHRIFT

(11) **EP 2 979 713 B1**
(45) Veröffentlichungstag und Bekanntmachung des Hinweises auf die Patenterteilung: **09.10.2019**
(21) Anmeldenummer: 15175975.0
(22) Anmeldetag: 09.07.2015
(51) Int. Cl.: A61M 5/158

(54) **INSERTIONSVORRICHTUNG FÜR EIN INFUSIONSSET**
INSERTION DEVICE FOR AN INFUSION SET
DISPOSITIF D'INSERTION POUR UN ENSEMBLE D'INFUSION

(30) Priorität: 29.07.2014 CH 11572014
(43) Veröffentlichungstag der Anmeldung: 03.02.2016
(73) Patentinhaber: TecPharma Licensing AG, 3401 Burgdorf (CH)
(72) Erfinder: Hirschel, Jürg, 3007 Bern (CH); Bernhard, Mario, 3400 Burgdorf (CH)

(56) Entgegenhaltungen:
- EP-A1- 1 762 259
- EP-A1- 2 174 680
- EP-A1- 2 457 606
- WO-A1-2009/010399
- WO-A1-2009/103759
- WO-A1-2010/112521
- WO-A1-2012/134588
- WO-A1-2015/032741

## Beschreibung

Die vorliegende Erfindung betrifft eine Insertionsvorrichtung für ein Infusionsset.

Infusionssets sind beispielsweise aus WO02/070037A2 und aus WO2005/049117A2 bekannt. Solche Infusionssets dienen beispielsweise der Insulinpumpentherapie, wobei Insulin von der Insulinpumpe mit Hilfe eines Infusionssets in den Körper eines Diabetikers transportiert wird. Das Infusionsset umfasst eine Kanüle, die vorzugsweise mittels einer Insertionsvorrichtung oder Einstechhilfen direkt unter, beziehungsweise in die Haut platziert und mit einem Pflaster an Ort und Stelle gehalten wird.

Eine Insertionsvorrichtung für ein solches Infusionsset ist beispielsweise aus EP1487519B1 bekannt. Nachteilig bei dieser Insertionsvorrichtung ist, dass bevor das Infusionsset in die Vorrichtung eingesetzt werden kann, eine Vortriebseinrichtung zur Insertion des Infusionssets von einer entspannten Position in eine betriebsbereite Position vorgespannt werden muss.

Weitere Insertionsvorrichtungen oder Einstechhilfen für ein Infusionsset sind aus EP1970084B1, EP1631337B1 und EP1970083A1 bekannt. Gemeinsam bei diesen Insertionsvorrichtungen ist, dass die Technik dieser Insertionsvorrichtungen auf einer Hebelmechanik basiert, wobei die Insertionsvorrichtungen folglich kompliziert aufgebaut sind und viele Bestandteile aufweisen.

Aus der WO2009/010399 A1, EP 2457606 A1, WO2010/112521 A1 und EP 1762259 A1 sind weitere Insertionsvorrichtungen für ein Infusionsset bekannt, wobei gemeinsam ist, dass ein Infusionsset mit einer Kanüle in die Insertionsvorrichtung eingesetzt wird, wobei mit Hilfe einer Nadel der Insertionsvorrichtung die Kanüle in die Haut des Patient gebracht wird.

Insbesondere ist aus der WO2009/010399 A1 eine Insertionsvorrichtung für ein Infusionsset bekannt, die eine Vortriebseinrichtung, ein Halteelement, das ein Führungselement aufweist, und weiter ein Basisteil, welches das Halteelement aufnimmt, wobei an dem Basisteil ein Führungsgegenelement angebracht ist, umfasst. Die Insertionsvorrichtung umfasst weiterhin ein Kopfteil, das relativ zu dem Basisteil um eine zentrale Achse drehbar angeordnet ist.

Es ist somit eine Aufgabe der vorliegenden Erfindung, eine Insertionsvorrichtung bereitzustellen, die einfacher zu bedienen ist und einen einfacheren Aufbau aufweist. Ferner ist eine Aufgabe der vorliegenden Erfindung, ein einfacheres und sicheres Verfahren zur Insertion eines Infusionssets mit einer solchen Insertionsvorrichtung bereitzustellen.

Diese Aufgabe wird durch den Gegenstand des unabhängigen Anspruchs gelöst. Vorteilhafte Ausführungsformen ergeben sich aus den abhängigen Ansprüchen.

Im Folgenden bedeutet proximale Position der Insertionsvorrichtung, eine Position, in welcher eine Vortriebseinrichtung zur Beförderung des Infusionsset in die Haut in einem gespannten Zustand ist, distale Position der Insertionsvorrichtung, eine Position, in welcher die Vortriebseinrichtung zur Beförderung des Infusionssets in die Haut in einem entspannten Zustand ist.

Die Erfindung betrifft eine Insertionsvorrichtung für ein Infusionsset mit einer Vortriebseinrichtung zum Bewegen des Infusionssets von einer proximalen Position in eine distale Position entlang einer zentralen Achse (z). Ferner weist die Insertionsvorrichtung ein Halteelement zum Halten des Infusionssets und ein Basisteil mit einer Kontaktfläche zum Aufsetzen der Insertionsvorrichtung auf eine Applikationsstelle am Körper eines Patienten auf, wobei das Basisteil von dem Halteelement aufgenommen ist. Zum Halten des Infusionssets weist das Halteelement mindestens einen, nicht-beanspruchten, Haltearm, vorzugsweise zwei oder ein Vielfaches von zwei Haltearmen auf. Der Haltearm ist derart ausgebildet, dass ein an dem Infusionsset vorgesehener Halteflügel in den Haltearm eingeschnappt und ausgeschnappt werden kann. Der Haltearm ist vorzugsweise elastisch ausgebildet. Die Insertionsvorrichtung umfasst des Weiteren ein Kopfteil, das relativ zu dem Basisteil um die zentrale Achse (z) drehbar angeordnet ist. Bei einer relativen Drehung zwischen dem Kopfteil und dem Basisteil in eine erste Drehrichtung ist ein an dem Halteelement angebrachtes Führungselement mit einem an dem Basisteil vorgesehenen Führungsgegenelement führbar, wobei das Führungselement des Halteelements mit dem am Basisteil vorgesehenen Führungsgegenelement in einem Führungseingriff ist, und wobei das Halteelement relativ zu dem Basisteil von einer distalen Position in eine proximale Position entlang der zentralen Achse (z) bewegbar ist.

Bei einer relativen Drehung zwischen dem Kopfteil und dem Basisteil in eine zweite Drehrichtung ist das Führungselement des Halteelements mit dem an dem Basisteil vorgesehenen Führungsgegenelement führbar, wobei das Halteelement relativ zu dem Basisteil von der proximalen Position in die distale Position entlang der zentralen Achse (z) bewegbar ist. In anderen Worten ausgedrückt, ist das Halteelement entlang der Achse (z) bewegbar, da das Führungselement des Halteelements mit dem am Basisteil vorgesehenen Führungsgegenelement in einem Führungseingriff ist.

Vorzugsweise ist das Führungselement des Halteelements als Führungskurve und das Führungsgegenelement des Basisteils als Führungsnocke ausgebildet. Alternativ kann das Führungselement des Halteelements als Führungsnocke und das Führungsgegenelement des Basisteils als Führungskurve ausgebildet sein. Es kann auch ein anderes Führungselement oder ein anderes Führungsgegenelement vorgesehen sein, vorausgesetzt, dass das Führungselement und das Führungsgegenelement im Führungseingriff sind.

Das Halteelement ist vorzugsweise drehfest mit dem Kopfteil verbunden. Das Kopfteil kann mit dem Halteelement relativ zu dem Basisteil in eine erste Drehrichtung und/oder zweite Drehrichtung drehen.

Zudem kann ein Deckel drehfest mit dem Kopfteil verbunden sein. Alternativ kann der Deckel einteilig mit dem Kopfteil ausgebildet sein. Vorzugsweise hat das Kopfteil an dessen proximalen Ende den Deckel aufgenommen.

Vorzugsweise kann die Vortriebseinrichtung ein elastisches Mittel und ein Gleitelement aufweisen. Bei einer relativen Drehung zwischen dem Kopfteil und dem Basisteil in die erste Drehrichtung ist das elastische Mittel spannbar, wobei bei einer relativen Drehung zwischen dem Kopfteil und dem Basisteil in die zweite Drehrichtung das elastische Mittel entspannbar ist. Das elastische Mittel kann an dem Gleitelement und an dem Kopfteil oder dem Deckel oder alternativ an dem Basisteil abgestützt sein. Das elastische Mittel kann somit als Druckfeder oder Zugfeder ausgebildet sein.

Vorzugsweise kann in der proximalen Position der Vortriebseinrichtung das Gleitelement von einer ersten Position in eine zweite Position drehbar sein, wobei in der ersten Position das Gleitelement von dem Halteelement haltbar ist und in der zweiten Position das Gleitelement von dem Halteelement freigebbar ist, sodass das Gleitelement entlang der zentralen Achse (z) relativ zu dem Halteelement axial bewegbar ist.

Das Gleitelement ist vorzugsweise relativ zu dem Haltelement in der distalen und in der proximalen Position der Vortriebseinrichtung um einen Drehwinkel, vorzugsweise um einen Drehwinkel von etwa 10°, versetzt zueinander angeordnet. Es kann auch ein anderer Drehwinkel vorgesehen sein.

Das Gleitelement kann ein Schlagelement zum Herausschlagen des Infusionssets aus dem Halteelement aufweisen oder als hülsenförmiges Schlagelement ausgebildet sein.

Vorzugsweise hat der Deckel einen Druckknopf aufgenommen. Der Druckknopf kann entlang der zentralen Achse (z) um einen Betätigungshub (H) verschiebbar sein. Ferner kann der Druckknopf derart ausgebildet sein, dass bei Betätigung des Druckknopfs um den Betätigungshub (H) das Gleitelement relativ zu dem Halteelement um die zentrale Achse (z) um den Drehwinkel in die zweite Position drehbar ist.

Es gibt ferner ein nicht-beanspruchtes Verfahren zur Insertion eines Infusionssets mit einer Insertionsvorrichtung nach einem der vorhergehenden Ansprüche mit den folgenden Schritten:
- Durchführung einer relativen Drehbewegung zwischen dem Kopfteil (5) und dem Basisteil (1) in eine zweite Drehrichtung
- Einlegen eines Infusionssets in ein Halteelement (2)
- Durchführung einer relativen Drehbewegung zwischen dem Kopfteil (5) und dem Basisteil (1) in eine erste Drehrichtung
- Aufsetzen der Insertionsvorrichtung auf eine Applikationsstelle am Körper eines Patienten
- Auslösen der Insertionsvorrichtung mittels Betätigung eines Deckels (6) oder eines Druckknopfes (7)
- Entfernen der Insertionsvorrichtung von der Applikationsstelle am Körper des Patienten.

Die Erfindung wird im Folgenden an mehreren Figuren beschrieben. Die hierbei offenbarten Merkmale bilden je einzeln und in Kombination die Erfindung vorteilhaft weiter. Es zeigen:
- Figur 1:: eine Explosionsansicht einer Ausführungsform einer Insertionsvorrichtung
- Figur 2a:: eine Längsschnittansicht der Ausführungsform der Insertionsvorrichtung in einer Anfangsposition, wobei die Längsschnittansicht der in Figur 2c eingezeichneten Schnittlinie A-A entspricht
- Figur 2b: eine Längsschnittansicht der Ausführungsform der Insertionsvorrichtung in der Anfangsposition gemäss Figur 2a, wobei die Längsschnittansicht der in Figur 2c eingezeichneten Schnittlinie B-B entspricht

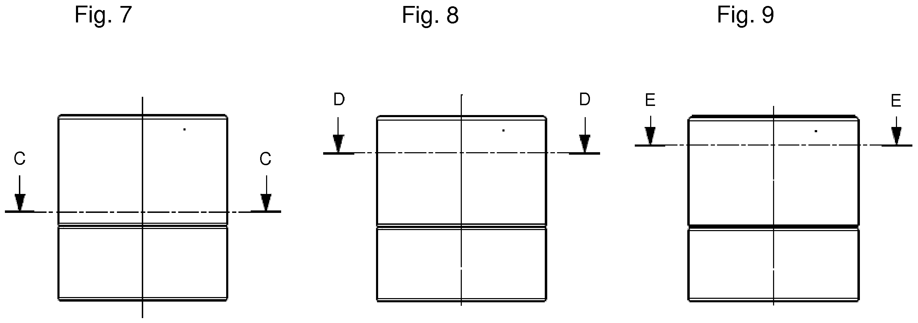

- Figur 2c: eine Querschnittansicht der Ausführungsform der Insertionsvorrichtung in der Anfangsposition gemäss Figur 2a, wobei die Querschnittansicht der in Figur 7 eingezeichneten Schnittlinie C-C entspricht
- Figur 2d: eine Detailansicht der Ausführungsform der Insertionsvorrichtung in der Anfangsposition gemäss Figur 2a, wobei das Haltelement (2) und das Basisteil (1) ersichtlich sind
- Figur 2e: eine Detailansicht der Ausführungsform der Insertionsvorrichtung in der Anfangsposition gemäss Figur 2a, wobei das Kopfteil (5), das Basisteil (1) und das Gleitelement (3) ersichtlich sind
- Figur 2f: eine Detailansicht der Ausführungsform der Insertionsvorrichtung in der Anfangsposition gemäss Figur 2a, wobei das Kopfteil (5), das Basisteil (1) und das Halteelement (2) ersichtlich sind
- Figur 3a:: eine Längsschnittansicht der Ausführungsform der Insertionsvorrichtung mit einem eingesetzten Infusionsset, wobei die Längsschnittansicht der in Figur 2c eingezeichneten Schnittlinie A-A entspricht
- Figur 3b:: eine Längsschnittansicht der Ausführungsform der Insertionsvorrichtung mit dem eingesetzten Infusionsset gemäss Figur 3a, wobei die Längsschnittansicht der in Figur 2c eingezeichneten Schnittlinie B-B entspricht
- Figur 4a:: eine Längsschnittansicht der Ausführungsform der Insertionsvorrichtung in einer geladenen Position, wobei die Längsschnittansicht der in Figur 4c eingezeichneten Schnittlinie A-A entspricht
- Figur 4b:: eine Längsschnittansicht der Ausführungsform der Insertionsvorrichtung in der geladenen Position gemäss Figur 4a, wobei die Längsschnittansicht der in Figur 4c eingezeichneten Schnittlinie B-B entspricht
- Figur 4c:: eine Querschnittansicht der Ausführungsform der Insertionsvorrichtung in der geladenen Position gemäss Figur 4a, wobei die Querschnittansicht der in Figur 8 eingezeichneten Schnittlinie D-D entspricht
- Figur 4d: eine Detailansicht der Ausführungsform der Insertionsvorrichtung in der geladenen Position gemäss Figur 4a, wobei das Halteelement (2) und das Basisteil (1) ersichtlich sind
- Figur 4e: eine Detailansicht der Ausführungsform der Insertionsvorrichtung in der geladenen Position gemäss Figur 4a, wobei das Kopfteil (5), das Basisteil (1) und das Halteelement (2) ersichtlich sind
- Figur 5a:: eine Längsschnittansicht der Ausführungsform der Insertionsvorrichtung in einer ausgelösten Position, wobei die Längsschnittansicht der in Figur 5c eingezeichneten Schnittlinie A-A entspricht
- Figur 5b:: eine Längsschnittansicht der Ausführungsform der Insertionsvorrichtung in der ausgelösten Position gemäss Figur 5a, wobei die Längsschnittansicht der in Figur 5c eingezeichneten Schnittlinie B-B entspricht
- Figur 5c: eine Querschnittansicht der Ausführungsform der Insertionsvorrichtung in der ausgelösten Position gemäss Figur 5a, wobei die Querschnittansicht der in Figur 9 eingezeichneten Schnittlinie E-E entspricht
- Figur 6a:: eine Längsschnittansicht der Ausführungsform der Insertionsvorrichtung mit einem in die Haut eines Patienten eingestochenen Infusionssets, wobei die Längsschnittansicht der in Figur 5c eingezeichneten Schnittlinie A-A entspricht
- Figur 6b:: eine Längsschnittansicht der Ausführungsform der Insertionsvorrichtung mit dem in die Haut eines Patienten eingestochenen Infusionssets gemäss Figur 6a, wobei die Längsschnittansicht der in Figur 5c eingezeichneten Schnittlinie B-B entspricht
- Figur 6c: eine Detailansicht der Ausführungsform der Insertionsvorrichtung mit dem in die Haut eines Patienten eingestochenen Infusionssets gemäss Figur 6a, wobei das Kopfteil (5), das Basisteil (1), das Gleitelement (3) und das Haltelement ersichtlich sind
- Figur 7: eine Darstellung der Insertionsvorrichtung mit eingezeichneter Schnittlinie C-C
- Figur 8: eine Darstellung der Insertionsvorrichtung mit eingezeichneter Schnittlinie D-D
- Figur 9: eine Darstellung der Insertionsvorrichtung mit eingezeichneter Schnittlinie E-E

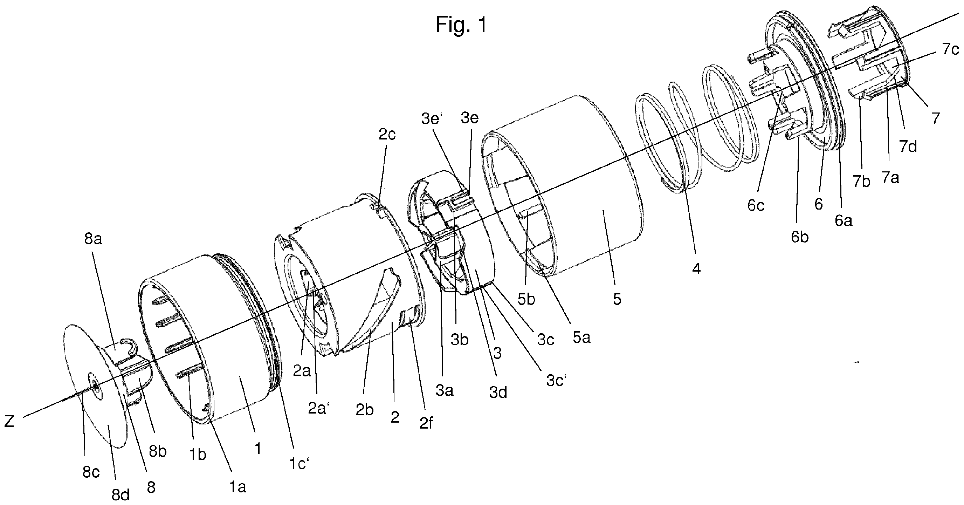

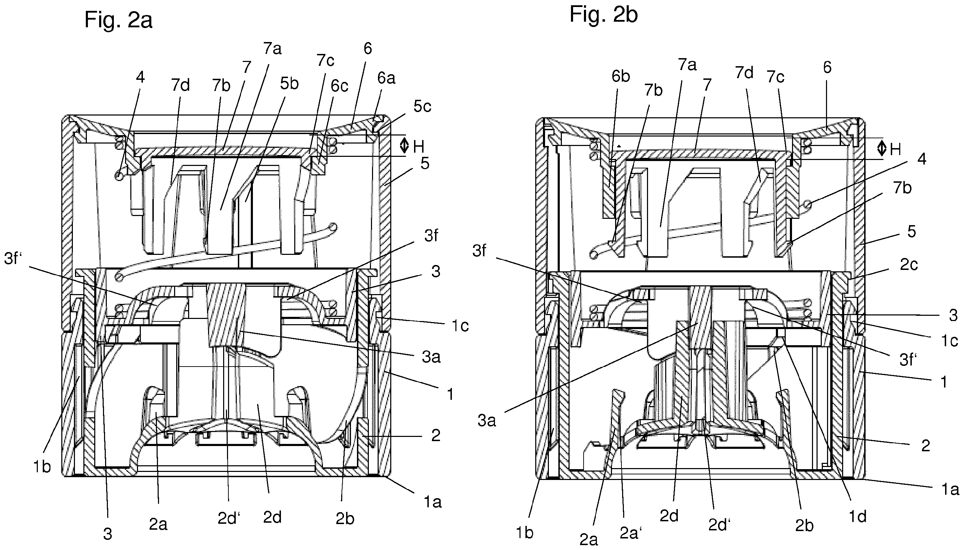

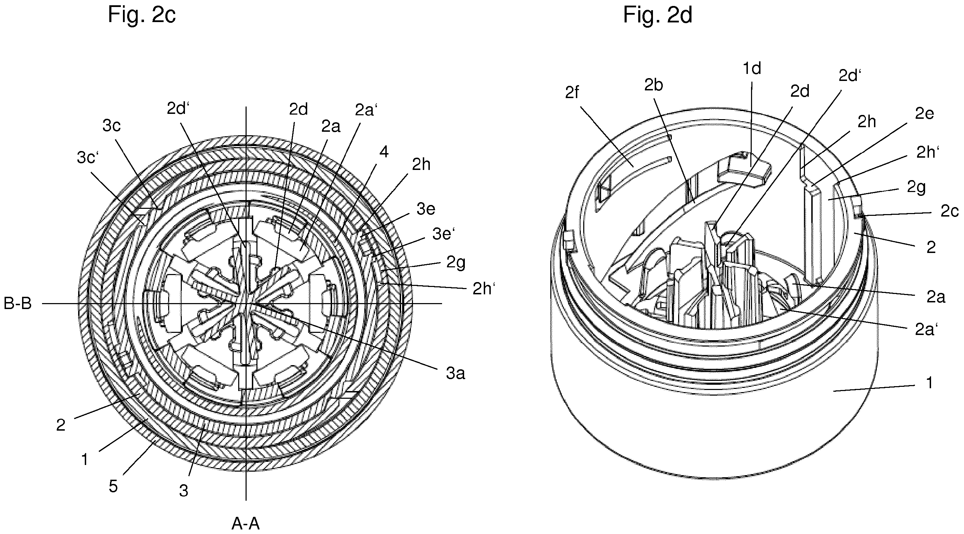

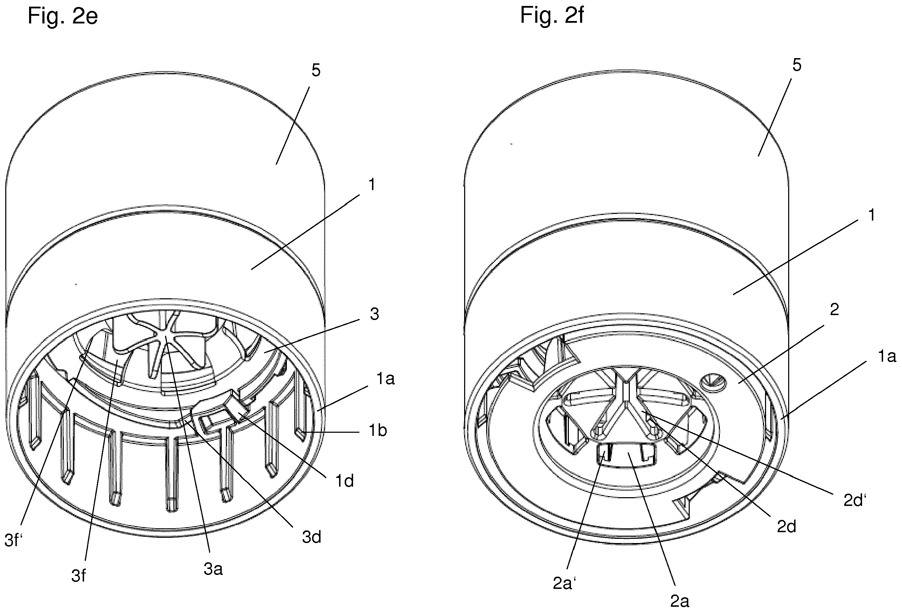

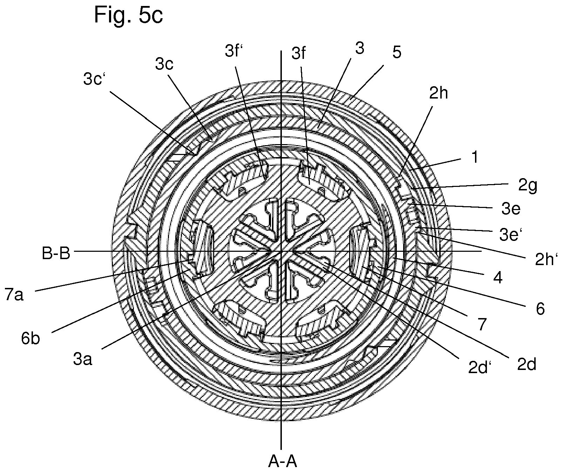

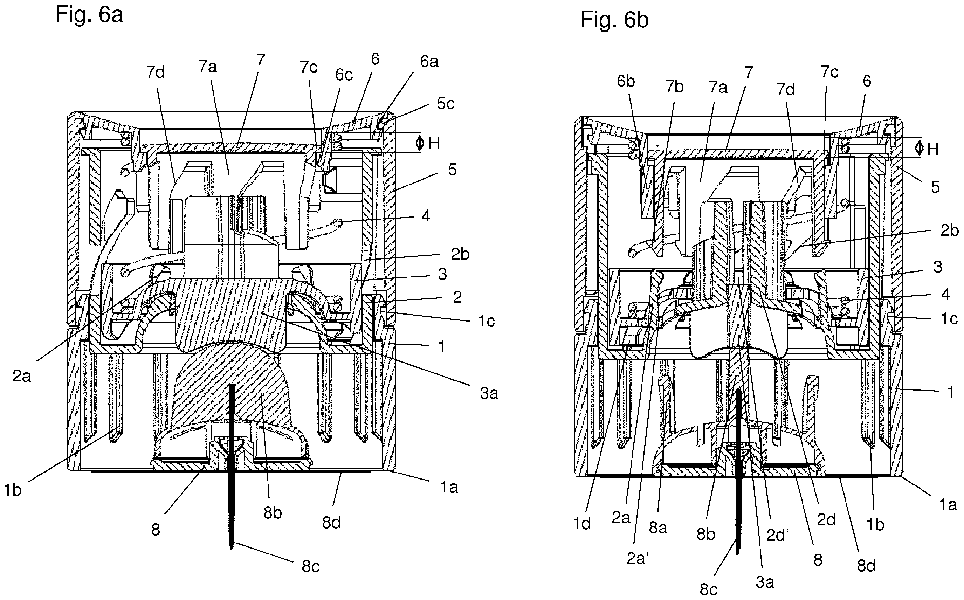

In der Figur 1 ist eine Explosionsansicht einer Ausführungsform einer erfindungsgemässen Insertionsvorrichtung dargestellt. Die Injektionsvorrichtung umfasst ein Halteelement (2) zum Halten eines Infusionssets (8), wobei das Infusionsset (8) eine Kanüle (8c) zum Einstechen in die Haut eines Patienten und ein Pflaster (8d) zum Befestigen des Infusionssets auf der Haut des Patienten umfassen kann. Dazu umfasst das Halteelement (2) mindestens zwei Haltearme (beispielsweise in Fig. 2a oder Fig. 2f, 2a), wobei die mindestens zwei elastischen Haltearme (beispielsweise in Fig. 2a oder Fig. 2f, 2a) jeweils radial um 180° versetzt zueinander angeordnet sind, oder eine Mehrzahl von zwei elastischen Haltearmen (beispielsweise in Fig. 2a oder Fig. 2f; 2a), in diesem Beispiel sechs Haltearme (beispielsweise in Fig. 2a oder Fig. 2f; 2a). Die Haltearme (beispielsweise in Fig. 2a oder Fig. 2f, 2a) sind derart ausgebildet, dass zwei an dem Infusionsset (8) angeordnete Halteflügel (8a) in die Haltearme (beispielsweise in Fig. 2a oder Fig. 2f, 2a) einschnappen und ausschnappen können. Das Halteelement (2) umfasst ferner mindestens zwei Halteschlitze (beispielsweise in Fig. 2b oder Fig. 2f, 2a'), wobei die mindestens zwei Halteschlitze (beispielsweise in Fig. 2b oder Fig. 2f, 2a') jeweils radial um 180° versetzt zueinander angeordnet sind, oder eine Mehrzahl von zwei Halteschlitze (beispielsweise in Fig. 2b oder Fig. 2f, 2a'), in diesem Beispiel sechs Halteschlitze (beispielsweise in Fig. 2b oder Fig. 2f, 2a'), durch welche die zwei an dem Infusionsset (8) vorgesehenen Halteflügel (8a) ein- und ausgeführt werden können. Damit das Infusionsset (8) in dem Halteelement (2) geführt wird, weist das Halteelement (2) am distalen Ende einen Führungsschlitz (beispielsweise in Fig. 2a oder Fig. 2f, 2d'), in diesem Beispiel drei Führungsschlitze (beispielsweise in Fig. 2a oder Fig. 2f, 2d'), auf. An dem Führungsschlitz (beispielsweise in Fig. 2a oder Fig. 2f, 2d') sind in proximaler Richtung ragende Führungsstege (beispielsweise in Fig. 2a oder Fig. 2d, 2d) vorgesehen, sodass eine an dem Infusionsset (8) angebrachte Haltelasche (8b) in das Halteelement (2) führbar ist. Der Führungssteg (beispielsweise in Fig. 2d, 2d) des Haltelements (2) kann verschiedene Längen aufweisen, wobei der Führungssteg (beispielsweise in Fig. 2d, 2d) oder ein Paar von Führungsstegen (beispielsweise in Fig. 2d, 2d) derart ausgebildet sein kann, dass eine Führung des Infusionsset (8) und eine relative Drehung zwischen dem Gleitelement (3) und dem Halteelement (2) gewährleistet werden kann. Ferner umfasst die Insertionsvorrichtung ein Kopfteil (5) und ein Basisteil (1), wobei das Basisteil (1) das Halteelement (2) aufgenommen hat. Das Basisteil (1) umfasst eine Kontaktfläche (1a) zum Aufsetzen der Insertionsvorrichtung auf eine Applikationsstelle am Körper des Patienten. An der Mantelinnenfläche des Basisteils (1) sind Längsstege (1b) zur axialen Führung des Halteelements (2) entlang einer zentralen Achse (z) angeordnet. An dem proximalen Ende und an der Mantelaussenfläche des Basisteils (1) ist eine Ringnut (1c) vorgesehen. An dem distalen Ende und an der Mantelinnenfläche des Kopfteils (5) ist ein Schnapper oder vorzugsweise mehrere Schnapper (5a), in diesem Beispiel mindestens vier Schnapper (5a) angebracht. Die Schnapper (5a) des Kopfteils (5) sind in die Ringnut (1c) des Basisteils (1) eingeschnappt, derart, dass das Kopfteil (5) relativ zu dem Basisteil (5) drehbar ist. Das Halteelement (1) weist ein Führungselement in Form einer Führungskurve (2b) auf, welche in Eingriff mit einem an dem Basisteil (1) vorgesehenen Führungsgegenelement in Form einer Führungsnocke (beispielsweise in Fig. 2b, 1d) ist. Bei einer relativen Drehung zwischen dem Kopfteil (5) und dem Basisteil (1) in eine erste Drehrichtung ist die Führungskurve (2b) des Haltelements (2) mit der an dem Basisteil (1) vorgesehener Führungsnocke (beispielsweise in Fig. 2b, 1d) führbar, wobei das Halteelement (2) relativ zu dem Basisteil (1) von einer distalen Position in eine proximale Position entlang der zentralen Achse (z) bewegbar ist. Dabei ist das Halteelement (2) aufgrund, beziehungsweise mittels der Führungspaare (1d, 2b) führbar. Bei einer relativen Drehung zwischen dem Kopfteil (5) und dem Basisteil (1) in eine zweite Drehrichtung ist die Führungskurve (2b) des Halteelements (2) ebenfalls mit der an dem Basisteil (1) vorgesehener Führungsnocke (beispielsweise in Fig. 2b, 1d) führbar, wobei das Halteelement (2) relativ zu dem Basisteil (1) von der proximalen Position in die distale Position entlang der zentralen Achse (z) bewegbar ist. Das Halteelement (2) ist mit dem Kopfteil (5) drehfest und axial bewegbar verbunden. Dazu umfasst das Halteelement (2) an der Mantelaussenfläche eine Nut (2c), welche in Eingriff mit einer an der Mantelinnenfläche des Kopfteils (5) vorgesehener Längsrippe (5b) ist. Die Insertionsvorrichtung weist ferner eine Vortriebseinrichtung auf. Die Vortriebseinrichtung umfasst ein elastisches Mittel in Form einer Druckfeder (4) und einem Gleitelement (3). Die Druckfeder (4) ist zwischen dem Gleitelement (3) und einem Deckel (6) spannbar gelagert. Der Deckel (6) ist über eine Schnappverbindung mit dem Kopfteil (5) axialfest verbunden. Dazu weist der Deckel (6) an dessen proximalen Ende eine Deckelnut (beispielsweise in Fig. 2a, 6a) auf, die mit einem am Kopfteil (5) vorgesehenen Ringsteg (beispielsweise in Fig. 2a, 5c) in Eingriff ist. Das Gleitelement (3) weist ein Schlagelement (3a) auf, um das Infusionsset (8) über die Haltelasche (8b) aus der Insertionsvorrichtung herauszuschlagen. Ferner ist an dem Gleitelement (3) ein Gleitelementanschlag (3b) vorgesehen, der in Anschlagkontakt mit einem an dem Halteelement (2) an der Mantelinnenfläche angebrachten Halteelementanschlag (beispielsweise in Fig. 2d, 2e) kommen kann. Durch den Anschlagkontakt zwischen Gleitelementanschlag (3b) und dem Halteelementanschlag (beispielsweise in Fig. 2d, 2e), ist das Gleitelement (3) mit dem Halteelement (2) axial fest verbunden. Damit das Gleitelement (3) nicht selbstständig ausser Anschlagkontakt mit dem Halteelement (2) gelangen kann, kann ein an der Mantelinnenfläche des Halteelements (2) vorgesehener Schnapper (2f) in eine an der Mantelaussenfläche des Gleitelements (3) vorgesehene erste Rille (3c) einschnappen. Zudem kann ein Anschlagkontakt zwischen einem an dem Gleitelement (3) vorgesehenen ersten Gleitelementsteg (3e) und einem an dem Haltelement (2) angebrachten ersten Halteelementsteg (beispielsweise Fig. 2d, 2h) vorgesehen sein, um eine relative Drehung zwischen dem Gleitelement (3) und dem Halteelement (2) in eine erste Drehrichtung zu begrenzen. Um eine relative Drehung zwischen dem Gleitelement (3) und dem Halteelement (2) in eine zweite Drehrichtung zu begrenzen, ist ein Anschlagkontakt zwischen einem an dem Gleitelement (3) vorgesehenen zweiten Gleitelementsteg (3e') und einem an dem Halteelement (2) angebrachten zweiten Halteelementsteg (beispielsweise Fig. 2d, 2h) vorgesehen. Wenn der zweiten Gleitelementsteg (3e') mit dem zweiten Haltelementsteg (beispielsweise Fig. 2d, 2h) in Anschlagkontakt ist, ist das Gleitelement (3) mit dem Halteelement (2) ausser Anschlagkontakt, sodass das Gleitelement (3) relativ zu dem Halteelement (2) axial verschiebbar ist. Dazu weist das Halteelement (2) an der Mantelinnenfläche eine Führungsnut (beispielsweise in Fig. 2d, 2g) auf, wobei der Gleitelementanschlag (3b) mit dem ersten und zweiten Gleitelementsteg (3e, 3e') entlang der Führungsnut (2g) gleiten kann. Dabei kann der Schnapper (2f) des Haltelements (2) in einer zweiten Rille (3c') des Gleitelements (3) liegen, wobei die zweite Rille (3c') radial versetzt zu der ersten Rille (3c) des Gleitelements angeordnet ist. Das Gleitelement (3) weist ferner eine Steuerkurve (3d) auf, die mit der Führungsnocke (beispielsweise in Fig. 2b, 1d) des Basisteils (1) in Führungseingriff gelangen kann. Bei einer relativen Drehung des Kopfteils (5) und des Basisteils (1), kann das Gleitelement (3) über die Führungsnocke (beispielsweise in Fig. 2e, 1d) des Basisteils (1) und über die Steuerkurve (beispielsweise in Fig. 2e, 3d) des Gleitelements (3) relativ zu dem Halteelement (2) gedreht werden. Das Gleitelement (3) bewegt sich relativ zu dem Halteelement (2) von einer ersten zu einer zweiten Position. Die Führungskurve (2b) des Halteelements (2), die Führungsnocke (beispielsweise in Fig. 2b, 1d) des Basisteils (1) und die Steuerkurve (beispielsweise in Fig. 2e, 3d) des Gleitelements (3) sind derart ausgebildet und angeordnet, dass das Gleitelement (3) relativ zu dem Halteelement (2) gedreht werden kann. Der Drehwinkel zwischen dem Gleitelement (3) und dem Halteelement (2) kann vorzugsweise etwa 10° sein. Es können auch andere Drehwinkel vorgesehen sein, beispielsweise etwa 12° oder 15°. Die Insertionsvorrichtung umfasst ferner einen Druckknopf (7), der von dem Deckel (6) aufgenommen ist. Der Druckknopf (7) weist einen in die distale Richtung ragenden Schnapparm (7a), in diesem Beispiel sechs Schnapparme (7a), auf. Die Schnapparme (7a) sind in Gleitkontakt mit an dem Deckel (6) vorgesehene Deckelstege (6b). Ein an dem jeweiligen Schnapparm (7a) angebrachter Schnapphaken (7b) bildet mit dem distalen Ende des Deckelstegs (6b) einen Anschlag, sodass die relative axiale Bewegung in die proximale Richtung des Druckknopfs (7) relativ zu dem Deckel (6) begrenzt wird. Die relative axiale Bewegung in die distale Richtung des Druckknopfs (7) relativ zu dem Deckel (6) wird zwischen einer am proximalen Ende des Druckknopfs (7) vorgesehener Druckknopfscheibe (7c) und einem am proximalen Ende des Deckels (6) angebrachter Deckelabsatz (6c) gebildet. Der Druckknopf (7) umfasst ferner eine Druckknopfschräge (7d), welche mit einer an einer Gleitelementöffnung (beispielsweise in Fig. 2e, 3f) vorgesehener Gleitelementkante (beispielsweise in Fig. 2e, 3f') des Gleitelements (3) zusammenwirken kann, um den Druckknopf (7) relativ zu dem Deckel (6) axial zu bewegen.

In der Figur 2a ist eine Längsschnittansicht der Ausführungsform der Insertionsvorrichtung in einer Anfangsposition, wobei die Längsschnittansicht der in Figur 2c eingezeichneten Schnittlinie A-A entspricht, dargestellt. Figur 2b zeigt die Insertionsvorrichtung gemäss Figur 2a, wobei die Längsschnittansicht der in Figur 2c eingezeichneten Schnittlinie B-B entspricht. Das Halteelement (2), der Druckknopf (7) und die Vortriebseinrichtung (3, 4) befinden sich in der distalen Position, wobei noch kein Infusionsset in der Insertionsvorrichtung eingesetzt ist. Wie in Figur 2d ersichtlich ist, liegt die Führungsnocke (1d) des Basisteils (1) in Anschlagkontakt mit dem proximalen Ende der Steuerkurve (2b) des Halteelements (2). Die Druckknopfscheibe (7c) des Druckknopfs (7) ist in Anschlagkontakt mit dem Deckelsteg (6b) des Deckels (6). Der Gleitelementanschlag (3b) des Gleitelements liegt auf dem Halteelementanschlag (2e) des Halteelements (2) auf, sodass das Gleitelement (3) mit dem Halteelement (2) axial fest verbunden ist. Der Schnapper (2f) des Halteelements (2) ragt in die erste Rille (3c) des Gleitelements (3), sodass das Gleitelement (3) nicht selbständig ausser Anschlagkontakt mit dem Halteelement (2) gelangen kann. Die Druckfeder (4) ist zwischen dem Deckel (6) und dem Gleitelement (3) gelagert, derart, dass das Gleitelement (3) und das Halteelement (2) in der distalen Position gehalten werden. Die Druckfeder (4) kann entspannt oder vorzugsweise vorgespannt sein. Der Schnapparm (7a) des Druckknopfs (7) ragt durch die Gleitelementöffnung (3f) des Gleitelements (3). Die Druckknopfscheibe (7a) des Druckknopfs (7) liegt auf dem Deckelabsatz (6c) des Deckels (6) auf.

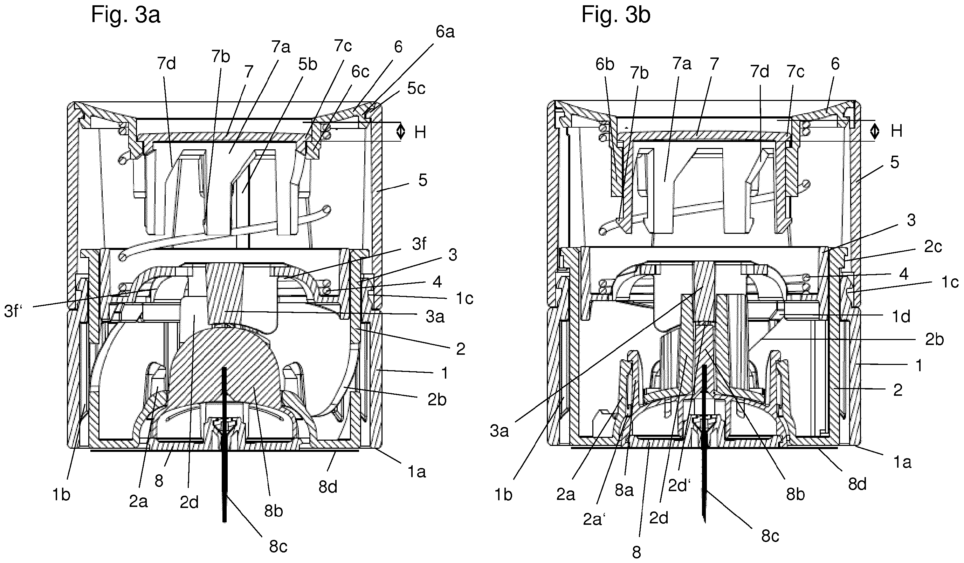

In der Figur 3a ist eine Längsschnittansicht der Ausführungsform der Insertionsvorrichtung mit dem eingesetzten Infusionsset, wobei die Längsschnittansicht der in Figur 2c eingezeichneten Schnittlinie A-A entspricht, dargestellt. Figur 3b zeigt die Insertionsvorrichtung gemäss Figur 3a, wobei die Längsschnittansicht der in Figur 2c eingezeichneten Schnittlinie B-B entspricht. Der Patient führt das Infusionsset (8) mit der Haltelasche (8b) durch den Führungsschlitz (2d') des Halteelements (2), wobei die Haltelasche (8b) durch die Führungsstege (2d) des Halteelements (2) geführt wird, sodass das Infusionsset (8) in die Insertionsvorrichtung eingeführt werden kann. Die Halteflügel (8a) des Infusionssets (8) durchdringen die Halteschlitze (2a') des Halteelements (2) und schnappen in die Haltearme (8a) des Halteelements (2) ein. Das Infusionsset (8) ist in der Insertionsvorrichtung eingesetzt. Das Gleitelement (3), insbesondere das Schlagelement (3a) des Gleitelements (3), und das Infusionsset (8), insbesondere die Haltelasche (8b) des Infusionssets (8), können so in Anschlagkontakt gelangen oder bevorzugt nicht in Anschlagkontakt gelangen, also einen Abstand entlang der zentralen Achse (z) aufweisen.

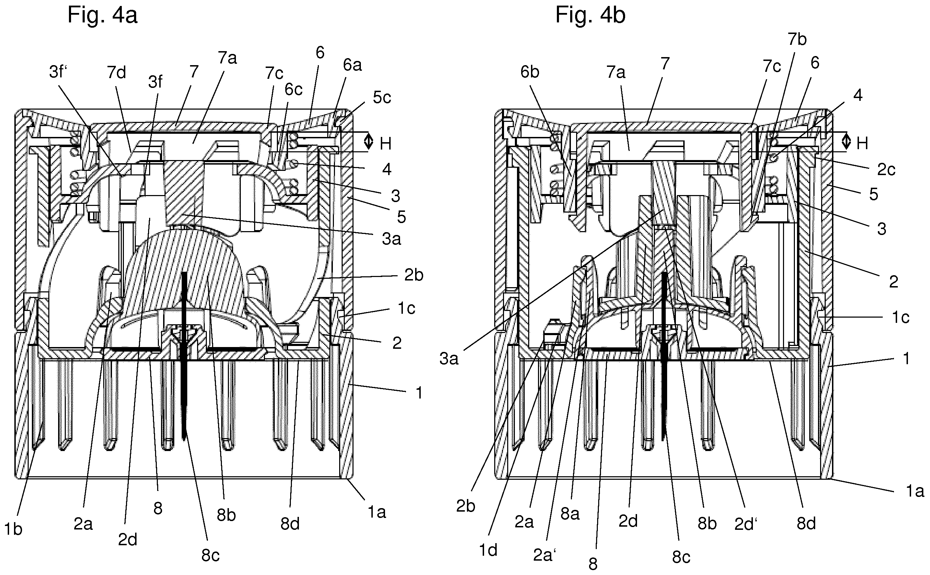

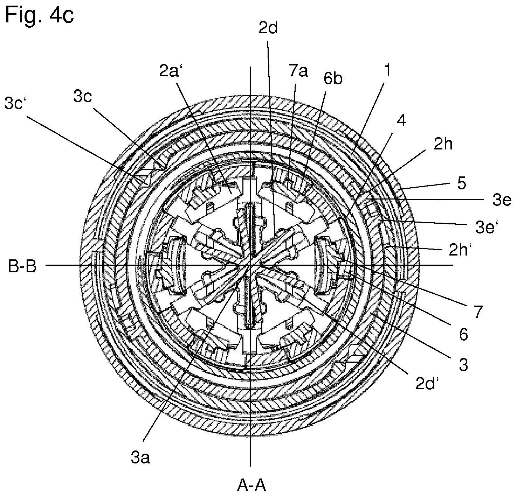

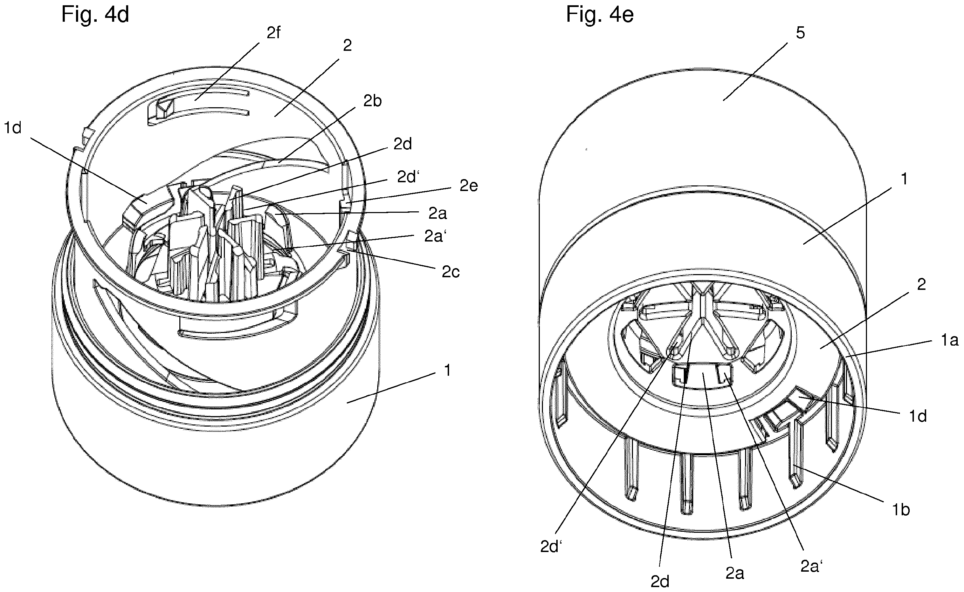

In der Figur 4a ist eine Längsschnittansicht der Ausführungsform der Insertionsvorrichtung in einer geladenen Position, wobei die Längsschnittansicht der in Figur 4c eingezeichneten Schnittlinie A-A entspricht, dargestellt. Figur 4b zeigt die Insertionsvorrichtung gemäss Figur 4a, wobei die Längsschnittansicht der in Figur 4c eingezeichneten Schnittlinie B-B entspricht. Der Patient dreht das Kopfteil (5) relativ zu dem Basisteil (1) der Insertionsvorrichtung in die erste Drehrichtung. Die Führungskurve (2b) des Halteelements (2) wird mit dem an dem Basisteil (1) angeordneten Führungsnocken (1d) geführt, sodass das Halteelement (2) relativ zu dem Basisteil (1) von der distalen Position in die proximale Position entlang der zentralen Achse (z) bewegt wird. Wie in Figur 4d und Figur 4e ersichtlich ist, kommt die Führungsnocke (1d) des Basisteils (1) in Anschlagkontakt mit dem distalen Ende der Steuerkurve (2b) des Halteelements (2) zu liegen. Das distale Ende der Steuerkurve (2b) des Haltelements (2) und die Führungsnocke (1d) des Basisteils (1) sind derart ausgebildet, dass die Führungsnocke (1d) des Basisteils (1) das Halteelement (2) in der proximalen Position halten kann. Der Gleitelementanschlag (3b) des Gleitelements (3) liegt auf dem Halteelementanschlag (2e) des Halteelements (2) auf, sodass das Gleitelement (3) mit dem Halteelement (2) in die proximale Position bewegt wird. Das Gleitelement (3) nimmt den Druckknopf (7) über die Gleitelementkante (3f') des Gleitelements (3) und über die Druckknopfschräge (7d) des Druckknopfs mit. Der Schnapphaken (7b) des Druckknopfs (7) gelangt in Anschlagkontakt mit dem distalen Endes des Deckelstegs (6b) des Deckels (6). Die Feder (4), welche zwischen dem Gleitelement (3) und dem Deckel (6) gelagert ist, wird dabei gespannt, beziehungsweise weitergespannt.

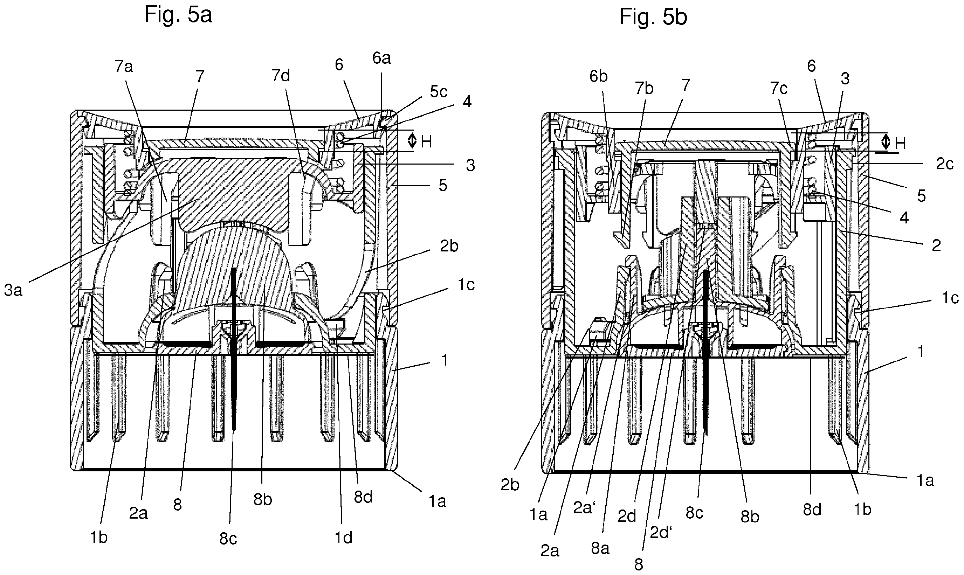

In der Figur 5a ist eine Längsschnittansicht der Ausführungsform der Insertionsvorrichtung in einer ausgelösten Position, wobei die Längsschnittansicht der in Figur 5c eingezeichneten Schnittlinie A-A entspricht, dargestellt. Figur 5b zeigt die Insertionsvorrichtung gemäss Figur 5a, wobei die Längsschnittansicht der in Figur 5c eingezeichneten Schnittlinie B-B entspricht. Der Patient drückt den Druckknopf (7) in die distale Richtung. Die Druckknopfschräge (7d) des Druckknopfes (7) wirkt mit der Gleitelementkante (3f') der Gleitelementöffnung (3f) des Gleitelements (3) derart zusammen, dass das Gleitelement (3) relativ zu dem Halteelement (2) gedreht wird, vorzugsweise um den Drehwinkel von etwa 10°. In den Figuren 2c, 4c und 5c ist die Drehwinkelverschiebung zwischen dem Gleitelement (3) und dem Haltelement (2) in der Anfangsposition, beziehungsweise der geladenen Position der Insertionsvorrichtung und der ausgelösten Position der Insertionsvorrichtung dargestellt. Der Schnapper (2f) des Halteelements (2) kommt beim Auslösen der Insertionsvorrichtung in die zweite Rille (3c') des Gleitelements (3) zu liegen. Der Gleitelementanschlag (3b) des Gleitelements (3) gelangt ausser Anschlagkontakt mit dem Haltelementanschlag (2e) des Haltelements (2). Der erste und zweite Gleitelementsteg (3e, 3e') des Gleitelements sind im Führungseingriff mit der Führungsnut (2g) des Halteelements (2), derart, dass das Gleitelement (3) über die Federkraft der gespannten Feder (4) relativ zu dem Haltelement (2) bewegt werden kann. Dabei schlägt das Schlagelement (3a) des Gleitelements (3) mit Hilfe der Federkraft der Feder (4) auf die Haltelasche (8b) des Infusionssets (8) auf, beziehungsweise drückt auf diese Haltelasche (8b), sodass die Halteflügel (8a) des Infusionssets (8) aus den Haltearmen (2a) des Halteelements (2) schnappen, das Infusionsset (8) nach distal bewegt wird und dadurch das Infusionsset mit der an dem Infusionsset vorgesehenen Kanüle (8c) in die Haut des Patienten eingestochen werden kann.

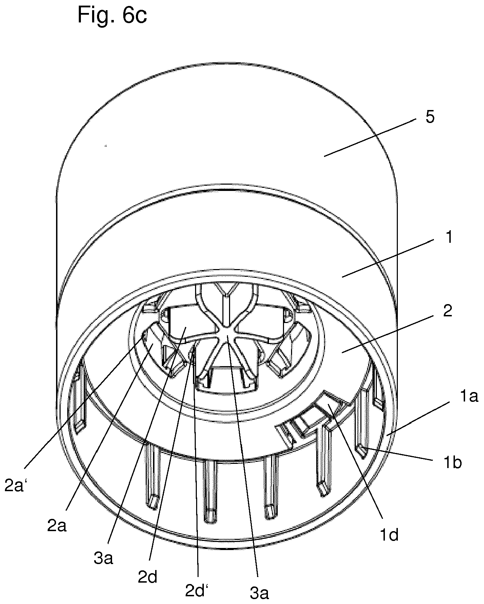

In der Figur 6a ist eine Längsschnittansicht der Ausführungsform der Insertionsvorrichtung mit einem in die Haut des Patienten eingestochenen Infusionssets, wobei die Längsschnittansicht der in Figur 6c eingezeichneten Schnittlinie A-A entspricht, dargestellt. Figur 6b zeigt die Insertionsvorrichtung gemäss Figur 6a, wobei die Längsschnittansicht der in Figur 6c eingezeichneten Schnittlinie B-B entspricht. Die Kanüle (8c) des Infusionssets (8) ist in die Haut des Patienten eingestochen. Die Druckknopfscheibe (7c) des Druckknopfs (7) ist in Anschlagkontakt mit dem Deckelabsatz (6c) des Deckels (6). Das Gleitelement (3) gelangt in Anschlagkontakt mit der Führungsnocke (1d) des Basisteils (1). Das Schlagelement (3a) des Gleitelements (3) ist ausser Anschlagkontakt mit der Haltelasche (8b) des Infusionssets (8). Wie in Figur 6c ersichtlich ist, ragt das Schlagelement (3a) des Gleitelements (3) durch die Führungsschlitze (2d') des Haltelements (2). Die Feder (4), die zwischen dem Deckel (6) und dem Gleitelement (3) gelagert ist, ist entspannt. Die Insertionsvorrichtung kann nun von der Haut des Patienten weggenommen werden, wobei das Infusionsset (8) über das an dem Infusionsset vorgesehene Pflaster (8d) auf der Haut des Patienten verbleibt.

Um die Insertionsvorrichtung wieder in die Anfangsposition gemäss Figur 2a und Figur 2b zu bringen, dreht der Patient das Kopfteil (5) relativ zu dem Basisteil (1) der Insertionsvorrichtung in die zweite Drehrichtung. Die Führungskurve (2b) des Halteelements (2) wird mit dem an dem Basisteil (1) angeordneten Führungsnocke (1d) geführt, sodass das Halteelement (2) relativ zu dem Basisteil (1) von der proximalen Position in die distale Position entlang der zentralen Achse (z) bewegt wird. Die Führungsnocke (1d) des Basisteils (1) gelangt in Anschlagkontakt mit dem proximalen Ende der Führungskurve (2b) des Halteelements (2). Das proximale Ende der Führungskurve (2b) des Haltelements (2), die Führungsnocke (1d) des Basisteils (1) und die Steuerkurve (3d) des Gleitelements (3) sind derart ausgebildet und angeordnet, dass das Gleitelement (3) relativ zu dem Halteelement (2) gedreht werden kann, vorzugsweise um den Drehwinkel von etwa 10°. Das Halteelement (2) befindet sich in der distalen Position, sodass ein neues Infusionsset (8) in die Insertionsvorrichtung eingesetzt werden kann.

### Bezugszeichen:

- 1: Basisteil
- 1a: Kontaktfläche
- 1b: Längssteg
- 1c: Ringnut
- 1d: Führungsnocke
- 2: Halteelement
- 2a: Haltearm
- 2a': Halteschlitz
- 2b: Führungskurve
- 2c: Nut
- 2d: Führungssteg
- 2d': Führungsschlitz
- 2e: Halteelementanschlag
- 2f: Schnapper
- 2g: Führungsnut
- 2h: erster Halteelementsteg
- 2h': zweiter Halteelementsteg
- 3: Gleitelement
- 3a: Schlagelement
- 3b: Gleitelementanschlag
- 3c: erste Rille
- 3c': zweite Rille
- 3d: Steuerkurve
- 3e: erster Gleitelementsteg
- 3e': zweiter Gleitelementsteg
- 3f: Gleitelementöffnung
- 3f': Gleitelementkante
- 4: Druckfeder
- 5: Kopfteil
- 5a: Schnapper
- 5b: Längsrippe
- 5c: Ringsteg
- 6: Deckel
- 6a: Deckelnut
- 6b: Deckelsteg
- 6c: Deckelabsatz
- 7: Druckknopf
- 7a: Schnapparm
- 7b: Schnapphaken
- 7c: Druckknopfscheibe
- 7d: Druckknopfschräge
- 8: Infusionsset
- 8a: Halteflügel
- 8b: Haltelasche
- 8c: Kanüle
- 8d: Pflaster
- z: zentrale Achse
- H: Betätigungshub

## Patentansprüche

1. Insertionsvorrichtung für ein Infusionsset (8) umfassend:
- eine Vortriebseinrichtung (3, 4) zum Bewegen des Infusionssets (8) von einer proximalen Position in eine distale Position entlang einer zentralen Achse (z),
- ein Halteelement (2) zum Halten des Infusionssets (8), wobei das Halteelement (2) ein Führungselement aufweist,
- ein Basisteil (1), welches das Halteelement (2) aufnimmt, wobei an dem Basisteil (1) ein Führungsgegenelement angebracht ist, und das Basisteil (1) eine Kontaktfläche (1a) zum Aufsetzen der Insertionsvorrichtung auf eine Applikationsstelle am Körper eines Patienten aufweist,
- ein Kopfteil (5), das relativ zu dem Basisteil (1) um die zentrale Achse (z) drehbar angeordnet ist,
wobei bei einer relativen Drehung zwischen dem Kopfteil (5) und dem Basisteil (1) in eine erste Drehrichtung das Führungselement des Halteelements (2) mit dem an dem Basisteil (1) vorgesehenen Führungsgegenelement führbar ist, wobei das Führungselement des Halteelements (2) mit dem am Basisteil (1) vorgesehenen Führungsgegenelement in einem Führungseingriff ist und das Halteelement (2) relativ zu dem Basisteil (1) von einer distalen Position in eine proximale Position entlang der zentralen Achse (z) bewegbar ist.

2. Insertionsvorrichtung nach Anspruch 1, **dadurch gekennzeichnet, dass** bei einer relativen Drehung zwischen dem Kopfteil (5) und dem Basisteil (1) in eine zweite Drehrichtung das Führungselement des Halteelements (2) mit dem an dem Basisteil (1) vorgesehenen Führungsgegenelement führbar ist, wobei das Halteelement (2) relativ zu dem Basisteil (1) von der proximalen Position in die distale Position entlang der zentralen Achse (z) bewegbar ist.

3. Insertionsvorrichtung nach Anspruch 1 oder 2, **dadurch gekennzeichnet, dass** das Führungselement des Halteelements (2) als Führungskurve (2b) und das Führungsgegenelement des Basisteils (1) als Führungsnocke (1d) ausgebildet ist.

4. Insertionsvorrichtung nach einem der vorhergehenden Ansprüche, **dadurch gekennzeichnet, dass** das Halteelement (2) drehfest mit dem Kopfteil (5) verbunden ist.

5. Insertionsvorrichtung nach einem der vorhergehenden Ansprüche, **dadurch gekennzeichnet, dass** ein Deckel (6) drehfest mit dem Kopfteil (5) verbunden ist.

6. Insertionsvorrichtung nach einem der vorhergehenden Ansprüche, **dadurch gekennzeichnet, dass** die Vortriebseinrichtung (3, 4) ein elastisches Mittel (4) und ein Gleitelement (3) aufweist.

7. Insertionsvorrichtung nach Anspruch 6, **dadurch gekennzeichnet, dass** bei der relativer Drehung zwischen dem Kopfteil (5) und dem Basisteil (1) in die erste Drehrichtung das elastische Mittel (4) spannbar ist.

8. Insertionsvorrichtung nach Anspruch 6 oder 7, **dadurch gekennzeichnet, dass** das elastische Mittel (4) an dem Gleitelement (3) und dem Kopfteil (5) oder dem Deckel (6) oder dem Basisteil (1) abgestützt ist.

9. Insertionsvorrichtung nach einem der Ansprüche 6 bis 8, **dadurch gekennzeichnet, dass** das elastische Mittel (4) als eine Druckfeder oder als eine Zugfeder ausgebildet ist.

10. Insertionsvorrichtung nach einem der Ansprüche 6 bis 8, **dadurch gekennzeichnet, dass** in der proximalen Position der Vortriebseinrichtung das Gleitelement (3) von einer ersten Position in eine zweite Position drehbar ist, wobei in der ersten Position das Gleitelement (3) von dem Halteelement (2) haltbar ist und in der zweiten Position das Gleitelement (3) von dem Halteelement (2) freigebbar ist, sodass das Gleitelement (3) entlang der zentralen Achse (z) relativ zu dem Halteelement (2) axial bewegbar ist.

11. Insertionsvorrichtung nach einem der Ansprüche 6 bis 10, **dadurch gekennzeichnet, dass** das Gleitelement (3) relativ zu dem Haltelement (2) in der distalen und in der proximalen Position der Vortriebseinrichtung um einen Drehwinkel, vorzugsweise um einen Drehwinkel von etwa 10°, versetzt zueinander angeordnet ist.

12. Insertionsvorrichtung nach einem der Ansprüche 6 bis 11, **dadurch gekennzeichnet, dass** das Gleitelement (3) ein Schlagelement zum Herausschlagen des Infusionssets (8) aus dem Halteelement (2) aufweist.

13. Insertionsvorrichtung nach Anspruch 5 bis 12, **dadurch gekennzeichnet, dass** das Kopfteil (5) an dessen proximalen Ende den Deckel (6) aufgenommen hat.

14. Insertionsvorrichtung nach Anspruch 5 bis 13, **dadurch gekennzeichnet, dass** der Deckel (6) einen Druckknopf (7) aufgenommen hat.

15. Insertionsvorrichtung nach Anspruch 14, **dadurch gekennzeichnet, dass** der Druckknopf (7) entlang der zentralen Achse (z) um einen Betätigungshub (H) verschiebbar ist.

16. Insertionsvorrichtung nach Anspruch 15, **dadurch gekennzeichnet, dass** der Druckknopf (7) derart ausgebildet ist, dass bei Betätigung des Druckknopfs (7) um den Betätigungshub (H) das Gleitelement (3) relativ zu dem Halteelement (2) um die zentrale Achse (z) in die zweite Position drehbar ist.

## Claims

1. An insertion device for an infusion set (8) including:
- an advance unit (3, 4) for moving the infusion set (8) along a central axis (z) from a proximal position into a distal position,
- a holding element (2) for holding the infusion set (8), wherein the holding element (2) has a guide element,
- a base portion (1) which receives the holding element (2), wherein a counterpart guide element is mounted to the base portion (1) and the base portion (1) has a contact surface (1a) for fitting the insertion device on to an application point on the body of a patient,
- a head portion (5) arranged rotatably about the central axis (z) relative to the base portion (1),
wherein upon a relative rotation between the head portion (5) and the base portion (1) in a first direction of rotation the guide element of the holding element (2) can be guided with the counterpart guide element provided on the base portion (1), wherein the guide element of the holding element (2) is in guiding engagement with the counterpart guide element provided on the base portion (1) and the holding element (2) is moveable along the central axis (z) relative to the base portion (1) from a distal position into a proximal position.

2. An insertion device according to claim 1 **characterised in that** upon a relative rotation between the head portion (5) and the base portion (1) in a second direction of rotation the guide element of the holding element (2) can be guided with the counterpart guide element provided on the base portion (1), wherein the holding element (2) is moveable along the central axis (z) relative to the base portion (1) from the proximal position into the distal position.

3. An insertion device according to claim 1 or claim 2 **characterised in that** the guide element of the holding element (2) is in the form of a curved guide track (2b) and the counterpart guide element of the base portion (1) is in the form of a guide projection (1d).

4. An insertion device according to one of the preceding claims **characterised in that** the holding element (2) is non-rotatably connected to the head portion (5).

5. An insertion device according to one of the preceding claims **characterised in that** a cover (6) is non-rotatably connected to the head portion (5).

6. An insertion device according to one of the preceding claims **characterised in that** the advance device (3, 4) has an elastic means (4) and a sliding element (3).

7. An insertion device according to claim 6 **characterised in that** the elastic means (4) can be tensioned upon the relative rotation between the head portion (5) and the base portion (1) in the first direction of rotation.

8. An insertion device according to claim 6 or claim 7 **characterised in that** the elastic means (4) is supported on the sliding element (3) and the head portion (5) or the cover (6) or the base portion (1).

9. An insertion device according to one of claims 6 to 8 **characterised in that** the elastic means (4) is in the form of a compression spring or in the form of a tension spring.

10. An insertion device according to one of claims 6 to 8 **characterised in that** in the proximal position of the advance device the sliding element (3) is rotatable from a first position into a second position, wherein the sliding element (2) can be held by the holding element (2) in the first position and the sliding element (3) can be released by the holding element (2) in the second position so that the sliding element (3) is axially moveable along the central axis (z) relative to the holding element (2).

11. An insertion device according to one of claims 6 to 10 **characterised in that** the sliding element (3) is arranged in mutually displaced relationship through a rotational angle, preferably a rotational angle of about 10°, relative to the holding element (2) in the distal and in the proximal positions of the advance device.

12. An insertion device according to one of claims 6 to 11 **characterised in that** the sliding element (3) has a striker element for striking the infusion set (8) out of the holding element (2).

13. An insertion device according to claims 5 to 12 **characterised in that** the head portion (5) has accommodated the cover (6) at the proximal end of the head portion.

14. An insertion device according to claims 5 to 13 **characterised in that** the cover (6) has accommodated a pushbutton (7).

15. An insertion device according to claim 14 **characterised in that** the pushbutton (7) is displaceable along the central axis (z) by an actuating stroke (H).

16. An insertion device according to claim 15 **characterised in that** the pushbutton (7) is so adapted that upon actuation of the pushbutton (7) by the actuating stroke (H) the sliding element (3) is rotatable relative to the holding element (2) about the central axis (z) into the second position.

## Revendications

1. Dispositif d'insertion pour un ensemble de perfusion (8), comprenant :
- un système de propulsion (3, 4) pour le déplacement de l'ensemble de perfusion (8) d'une position proximale à une position distale le long d'un axe central (z),
- un élément de retenue (2) pour retenir l'ensemble de perfusion (8), dans lequel l'élément de retenue (2) présente un élément de guidage,
- une partie de base (1) qui loge l'élément de retenue (2), dans lequel un contre-élément de guidage est disposé au niveau de la partie de base (1) et la partie de base (1) présente une surface de contact (1a) pour le placement du dispositif d'insertion à un endroit d'application au niveau du corps d'un patient,
- une partie de tête (5) qui est disposée rotative autour de l'axe central (z) par rapport à la partie de base (1),
dans lequel l'élément de guidage de l'élément de retenue (2) peut être guidé avec le contre-élément de guidage prévu au niveau de la partie de base (1) lors d'une rotation relative entre la partie de tête (5) et la partie de base (1) dans une première direction de rotation, dans lequel l'élément de guidage de l'élément de retenue (2) est en prise de guidage avec le contre-élément de guidage prévu au niveau de la partie de base (1) et l'élément de retenue (2) peut être déplacé par rapport à la partie de base (1) le long de l'axe central (z) d'une position distale à une position proximale.

2. Dispositif d'insertion selon la revendication 1, **caractérisé en ce que** l'élément de guidage de l'élément de retenue (2) peut être guidé avec le contre-élément de guidage prévu au niveau de la partie de base (1) lors d'une rotation relative entre la partie de tête (5) et la partie de base (1) dans une deuxième direction de rotation, dans lequel l'élément de retenue (2) peut être déplacé par rapport à la partie de base (1) le long de l'axe central (z) d'une position proximale à une position distale.

3. Dispositif d'insertion selon la revendication 1 ou 2, **caractérisé en ce que** l'élément de guidage de l'élément de retenue (2) est conçu sous la forme d'une courbe de guidage (2b) et le contre-élément de guidage de la partie de base (1), sous la forme d'une came de guidage (1d).

4. Dispositif d'insertion selon l'une quelconque des revendications précédentes, **caractérisé en ce que** l'élément de retenue (2) est relié solidaire en rotation à la partie de tête (5).

5. Dispositif d'insertion selon l'une quelconque des revendications précédentes, **caractérisé en ce qu'**un couvercle (6) est relié solidaire en rotation à la partie de tête (5).

6. Dispositif d'insertion selon l'une quelconque des revendications précédentes, **caractérisé en ce que** le système de propulsion (3, 4) présente un moyen élastique (4) et un élément coulissant (3).

7. Dispositif d'insertion selon la revendication 6, **caractérisé en ce que** le moyen élastique (4) peut être tendu lors de la rotation relative entre la partie de tête (5) et la partie de base (1) dans la première direction de rotation.

8. Dispositif d'insertion selon la revendication 6 ou 7, **caractérisé en ce que** le moyen élastique (4) est appuyé contre l'élément coulissant (3) et la partie de tête (5) ou le couvercle (6) ou la partie de base (1).

9. Dispositif d'insertion selon l'une quelconque des revendications 6 à 8, **caractérisé en ce que** le moyen élastique (4) est conçu sous la forme d'un ressort de pression ou sous la forme d'un ressort de traction.

10. Dispositif d'insertion selon l'une quelconque des revendications 6 à 8, **caractérisé en ce que** l'élément coulissant (3) est rotatif d'une première position à une deuxième position dans la position proximale du système de propulsion, dans lequel, dans la première position, l'élément coulissant (3) peut être retenu par l'élément de retenue (2) et, dans la deuxième position, l'élément coulissant (3) peut être libéré de l'élément de retenue (2) de telle sorte que l'élément coulissant (3) peut être déplacé axialement par rapport à l'élément de retenue (2) le long de l'axe central (z).

11. Dispositif d'insertion selon l'une quelconque des revendications 6 à 10, **caractérisé en ce que** l'élément coulissant (3) est disposé décalé par rapport à l'élément de retenue (2) d'un angle de rotation, de préférence d'un angle de rotation d'environ 10°, dans les positions distale et proximale du système de propulsion.

12. Dispositif d'insertion selon l'une quelconque des revendications 6 à 11, **caractérisé en ce que** l'élément coulissant (3) présente un élément de frappe pour faire sortir l'ensemble de perfusion (8) hors de l'élément de retenue (2).

13. Dispositif d'insertion selon les revendications 5 à 12, **caractérisé en ce que** la partie de tête (5) a logé le couvercle (6) à son extrémité proximale.

14. Dispositif d'insertion selon les revendications 5 à 13, **caractérisé en ce que** le couvercle (6) a logé un bouton-poussoir (7).

15. Dispositif d'insertion selon la revendication 14, **caractérisé en ce que** le bouton-poussoir (7) peut être coulissé le long de l'axe central (z) sur une course d'actionnement (H).

16. Dispositif d'insertion selon la revendication 15, **caractérisé en ce que** le bouton-poussoir (7) est conçu de telle sorte que, lors de l'actionnement du bouton-poussoir (7) sur la course d'actionnement (H), l'élément coulissant (3) peut être amené en rotation par rapport à l'élément de retenue (2) autour de l'axe central (z) jusqu'à la deuxième position.
